# EUROPÄISCHE PATENTANMELDUNG

(11) **EP 3 009 126 A1**
(43) Veröffentlichungstag der Anmeldung: **20.04.2016**
(21) Anmeldenummer: 14188811.5
(22) Anmeldetag: 14.10.2014
(51) Int. Cl.: A61K 9/00

(54) **Oromukosale Filmzubereitung**

(71) Anmelder: SapioTec GmbH, 97082 Würzburg (DE)
(72) Erfinder: Roewer, Norbert, 97082 Würzburg (DE); Broscheit, Jens, 97209 Würzburg (DE)
(74) Vertreter: Glawe, Delfs, Moll

(57) **Zusammenfassung**

Gegenstand der Erfindung ist eine oromukosale Filmzubereitung. Erfindungsgemäß ist vorgesehen, dass sie einen wirkstoffhaltigen Flächenbereich (3) und einen wirkstofffreien Flächenbereich (4) aufweist. Der wirkstofffreie Flächenbereich (4) kann als Applikationshilfe zur sicheren und einfachen Applikation der Filmzubereitung verwendet werden.

## Beschreibung

Die Erfindung betrifft eine oromukosale Filmzubereitung.

Die perorale Gabe von Arzneimitteln ist weit verbreitet. Bekannt sind beispielsweise Tabletten oder Kapseln, die in der Regel mit Flüssigkeit geschluckt werden.

Ebenfalls beispielsweise aus WO 2012/055947 A1 bekannt sind wirkstoffhaltige dünne Filme, die in den Mundraum gelegt werden, sich dort lösen bzw. zerfallen und den enthaltenen Wirkstoff freisetzen. Der Vorteil dieser Darreichungsform ist die Verabreichung ohne zusätzliche Flüssigkeit und ohne bzw. nur mit geringer Schluckbelastung.

Solche oromukosalen Filmzubereitungen können insbesondere bei Patientengruppen von Vorteil sein, die beispielsweise aufgrund ihres Alters (Kinder oder ältere Patienten), aufgrund von Schluckbeschwerden oder beispielsweise aufgrund psychischer Erkrankungen eine schlechte Compliance bei der Einnahme herkömmlicher oraler Darreichungsformen aufweisen.

Der vorliegenden Erfindung liegt die Aufgabe zu Grunde, eine oromukosale Filmzubereitung der eingangs genannten Art zur Verfügung zu stellen, deren Anwendung bzw. Darreichung weiter verbessert ist.

Gelöst wird diese Aufgabe durch eine oromukosale Filmzubereitung, die einen wirkstoffhaltigen Flächenbereich und einen wirkstofffreien Flächenbereich aufweist.

Zunächst seien einige im Rahmen der Erfindung verwendete Begriffe erläutert.

Eine oromukosale Filmzubereitung (auch orodispersibler Film oder Schmelzfilm genannt) weist eine Kombination aus wenigstens einem Träger Filmbildner) und wenigstens einem Wirkstoff auf. Diese Kombination wird zu einem dünnen Film geformt, der in die Mundhöhle gelegt wird, sich dort unter Feuchtigkeitseinfluss löst oder disintegriert und enthaltene Wirkstoffe freisetzt. Die Dicke eines solchen Films liegt bevorzugt unter 1 mm, weiter vorzugsweise unter 500 µm, typischerweise zwischen 100 und 200 µm. Diese Angaben sind die Dicke im trockenen Zustand.

Der wirkstoffhaltige Flächenbereich ist ein Bereich, der in üblicher und im Stand der Technik bekannter Weise einen oder mehrere Wirkstoffe enthält und diese in der Mundhöhle freisetzen kann. Erfindungsgemäß ist vorgesehen, dass die Filmzubereitung zusätzlich einen wirkstofffreien Flächenbereich aufweist. Wirkstofffrei bedeutet in diesem Zusammenhang, dass dieser Flächenbereich keine oder allenfalls geringe Mengen an Wirkstoffen aufweist.

Zweck dieses wirkstofffreien Flächenbereichs ist die Erleichterung der Handhabung der erfindungsgemäßen Filmzubereitung. Beim Einlegen in die Mundhöhle kann der Patient oder eine dritte Hilfsperson die Filmzubereitung am wirkstofffreien Flächenbereich anfassen und applizieren. Es wird so vermieden, dass der Patient bzw. die Hilfsperson beim Einlegen in die Mundhöhle bereits mit Wirkstoff in Berührung kommt. Insbesondere wird vermieden, dass beispielsweise durch Hautfeuchtigkeit an den Fingern bereits Wirkstoff aus der Filmzubereitung vorzeitig freigesetzt wird.

Bevorzugt nimmt der wirkstofffreie Flächenbereich einen Anteil von 10 bis 50%, vorzugsweise 10 bis 40%, weiter vorzugsweise 20 bis 30% der Gesamtfläche der Filmzubereitung ein. Auf diese Weise stellt der wirkstofffreie Flächenbereich eine hinreichend große Handhabe dar, andererseits ist sichergestellt, dass Wirkstoff aus einem genügend großen wirkstoffhaltigen Flächenbereich freigesetzt werden kann. Die Gesamtfläche der erfindungsgemäßen Zubereitung kann bevorzugt zwischen 1 und 10 cm² liegen.

Der wirkstoffhaltige Flächenbereich und der wirkstofffreie Flächenbereich können aus zwei unterschiedlichen Filmen bestehen, die aneinander gefügt sind. Unterschiedlich bedeutet in diesem Zusammenhang, dass sich die beiden Flächenbereiche zumindest durch ihren Wirkstoffgehalt unterscheiden. Das Aneinanderfügen kann dergestalt erfolgen, dass eine feste Verbindung zwischen den beiden Filmbereichen vorhanden ist, die sich bei der normalen Handhabung und Applikation der Filmzubereitung nicht löst. Der wirkstofffreie Flächenbereich wird bei dieser Variante der Erfindung in der Regel mit in die Mundhöhle eingelegt und zerfällt bzw. löst sich dort ebenso wie der wirkstoffhaltige Flächenbereich.

Alternativ ist es erfindungsgemäß möglich, die Verbindung so auszugestalten, dass bei der Applikation der Filmzubereitung nach dem Einlegen in die Mundhöhle der wirkstofffreie Flächenbereich abgetrennt, vorzugsweise abgerissen werden kann. Beispielsweise kann sich diese Verbindung durch die Feuchtigkeit in der Mundhöhle schnell lösen, so dass sich nach dem Einlegen der wirkstofffreie Flächenbereich schnell ablöst und entnommen werden kann. Ebenfalls ist es denkbar, den wirkstofffreien Flächenbereich nicht oder lediglich teilweise in die Mundhöhle einzuführen, so dass diese nicht durch die Finger der handhabenden Person kontaminiert werden kann.

Erfindungsgemäße Filmzubereitungen weisen Filmbildner auf. Es handelt sich dabei um Stoffe, die als Trägerstoffe Wirkstoffe aufnehmen können und Struktur, Form und Lösungsverhalten der Filmzubereitungen beeinflussen bzw. bestimmen. In zwei Varianten der Erfindung sind die Filmbildner der zwei unterschiedlichen Filme bzw. Flächenbereiche identisch oder verschieden.

Identische Filmbildner erleichtern in die Regel die Herstellung und bewirken, dass sich der nicht wirkstoffhaltige Flächenbereich, sofern er nach der Applikation in der Mundhöhle verbleibt, in der Regel ähnlich verhält, insbesondere ähnlich löst, wie der wirkstoffhaltige Flächenbereich.

Unterschiedliche Filmbildner können dann von Vorteil sein, wenn man dem nicht wirkstoffhaltigen Flächenbereich gezielt andere Eigenschaften verleihen möchte. Beispielsweise kann es erwünscht sein, dass sich dieser nicht wirkstoffhaltige Flächenbereich in der Mundhöhle schneller löst, während der wirkstoffhaltige Flächenbereich über einen längeren Zeitraum in der Mundhöhle verbleibt und dort Wirkstoff freisetzt.

Der nicht wirkstoffhaltige Flächenbereich kann alternativ aus einem weniger oder gegebenenfalls nicht im Milieu der Mundhöhle löslichen Polymermaterial gefertigt sein, wenn es vorgesehen ist, dass dieser im Zuge der Applikation von dem wirkstoffhaltigen Flächenbereich abgetrennt wird und nicht in der Mundhöhle verbleibt. In diesem Fall kann das Material des wirkstofffreien Flächenbereichs so gewählt werden, dass beispielsweise dessen Abtrennung im Zuge der Applikation erleichtert wird.

Erfindungsgemäß geeignete Filmbildner sind grundsätzlich aus dem Stand der Technik bekannt und dem Fachmann geläufig. Die Filmbildner können beispielsweise ausgewählt sein aus der Gruppe bestehend aus Cellulose, Cellulosederivaten, Acryl- und Methacrylsäurepolymeren, Polyvinylalkoholen, Polyvinylacetat, Polyvinylpyrrolidon und Derivaten, Polysacchariden und Polymeren auf Stärkebasis.

Beispielhaft genannt als Filmbildner seien Hydroxyethylcellulose, Hydroxypropylcellulose, Hydroxypropylmethylcellulose, Methylcellulose, Natrosol^{®}, Tylose^{®} H300, Klucel^{®}, Klucel^{®} E, Klucel^{®} L, Klucel^{®} J, Klucel^{®} G, Klucel^{®} M, Klucel^{®} H, Klucel^{®} EF, Klucel^{®} LF, Klucel^{®} JF, Klucel^{®} GF, Klucel^{®} MF, Klucel^{®} HF, Klucel^{®} EXF, Klucel^{®} LXF, Klucel^{®} JXF, Klucel^{®} GXF, Klucel^{®} MXF, Klucel^{®} HXF, Pharmacoat^{®} 603, Pharmacoat^{®} 606, Methocel^{®} E4M, Polyethylenglykol-Polyvinylalkohol-Copolymere, Kollicoat^{®} IR, Kollicoat^{®} protect, Natriumcarboxymethylcellulose, Polysaccharide aus Maltotriose, Pullulan^{®}, Lycoat^{®} RS720, Lycoat^{®} RS780, Lycoat^{®} NG, und Hydroxyethylmethylcellulose.

Bevorzugt sind der wirkstoffhaltige Flächenbereich und der wirkstofffreie Flächenbereich für den Anwender unterscheidbar, bevorzugt optisch unterscheidbar, ausgebildet. Die Filmzubereitung kann so gezielt am wirkstofffreien Flächenbereich ergriffen werden. Die Unterscheidbarkeit kann beispielsweise bewirkt werden durch unterschiedliche Farben, optische Dichten, Markierungen, Strukturen oder Kombinationen daraus.

Gegenstand der Erfindung ist ferner ein Verfahren zur Herstellung einer erfindungsgemäßen Filmzubereitung. Erfindungsgemäß wird ein wirkstofffreier Film an einen wirkstoffhaltigen Film angefügt. Der Vorteil dieses Verfahrens ist es, dass ein wirkstoffhaltige Film in üblicher Weise hergestellt werden kann und erst nach Fertigstellung mit einem wirkstofffreien, ebenfalls separat hergestellten Film als Applikationshilfe zusammengefügt und damit kombiniert wird.

Das Anfügen kann erfindungsgemäß bevorzugt thermisch, durch ein Adhäsiv oder durch Feuchtigkeit erfolgen.

Wenn ein Film oder beide Filme thermoplastische Eigenschaften aufweisen, können diese beispielsweise erwärmt und im erwärmten Zustand zusammengefügt werden. Durch die thermoplastischen Eigenschaften entsteht eine für die Applikation hinreichend feste Verbindung.

Als Adhäsiv können geeignete Klebstoffe oder beispielsweise viskose Polymerlösungen verwendet werden, aus denen durch Trocknung Lösungsmittel abgezogen wird und die sich dadurch hinreichend verfestigen, um als Adhäsiv zu wirken.

Sofern Filmbildner von geeigneten Lösungsmitteln, insbesondere Wasser, gelöst oder angelöst werden können, kann ein Anfügen auch unter Feuchtigkeitseinwirkung erfolgen.

Bei einer alternativen Ausführungsform der Erfindung wird der wirkstofffreie Flächenbereich durch Filmbildung in situ an den wirkstoffhaltigen Film angefügt. Er kann beispielsweise an den fertigen wirkstoffhaltigen Film angegossen oder durch eine Extrusion oder Verpressung angefügt werden.

Ein Ausführungsbeispiel der Erfindung wird schematisch anhand der Zeichnung dargestellt. Darin zeigen:
- Fig. 1:: Schematisch die Herstellung eines erfindungsgemäßen Films durch Solvent-Casting;
- Fig. 2:: Eine Ansicht eines erfindungsgemäßen Films;
- Fig. 3:: Die orale Applikation eines erfindungsgemäßen Films.

Geeignete Beispielrezepturen für Polymerlösungen zur Herstellung eines erfindungsgemäßen Films sind wie folgt:

**Basislösung für den wirkstoffhaltigen Film:**

| | |
|---|---|
| Hydroxypropylmethylcellulose (Pharmacoat® 606) | 15 % |
| Glycerol, wasserfrei | 3 % |
| Kollidon® CL-M | 5 % |
| Wasser | 77 % |
| Kollidon® CL-M (BASF) ist ein sogenanntes Disintegrant, das durch Wasseraufnahme und Schwellen einen Film in wässriger Umgebung zerfallen lässt. | |

**Basislösung für den wirkstofffreien Film:**

| | |
|---|---|
| Kollicoat® IR Carmine | 25 % |
| Glycerol, wasserfrei | 1 % |
| Wasser | 74 % |

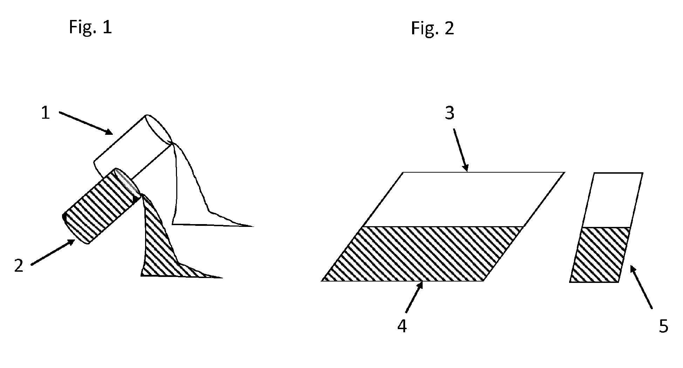

Fig. 1 zeigt schematisch, wie eine wirkstofffreie Polymerlösung 1 und eine wirkstoffhaltige Polymerlösung 2 gemeinsam vergossen werden (Solvent-Casting). Die beiden Polymerlösungen bzw. -schichten können entweder parallel oder nacheinander flächig ausgezogen werden und haften aneinander. Anschließend werden sie getrocknet. Die so hergestellte Filmzubereitung weist einen wirkstoffhaltigen Bereich 3 und einen wirkstofffreien Bereich 4 auf.

Eine so hergestellte Zubereitung kann in insgesamt mit 5 bezeichnete Streifen geeigneter Größe geschnitten und verpackt werden.

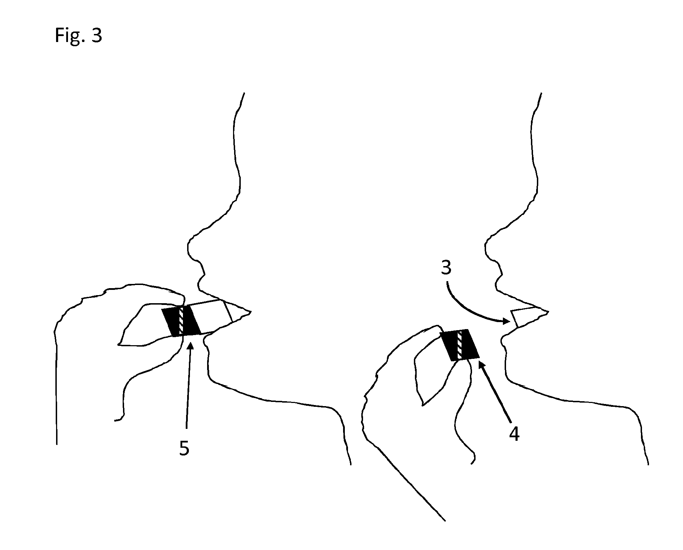

Fig. 3 zeigt schematisch die Anwendung. Der Patient entnimmt den Film an dem wirkstofffreien Bereich 4 aus der Verpackung und nimmt den wirkstoffhaltigen Bereich 3 in den Mund. Das hydrophile Polymer des Films beginnt sich durch den Kontakt mit dem Speichel zu lösen, so dass der wirkstofffreie Bereich 4 von dem wirkstoffhaltigen Bereich 3 abgelöst und entsorgt werden kann. Alternativ ist es ebenfalls möglich, die gesamte Filmzubereitung einschließlich des wirkstofffreien Bereichs 4 in den Mund zu nehmen.

## Patentansprüche

1. Oromukosale Filmzubereitung, **dadurch gekennzeichnet, dass** sie einen wirkstoffhaltigen Flächenbereich (3) und einen wirkstofffreien Flächenbereich (4) aufweist.

2. Filmzubereitung nach Anspruch 1, **dadurch gekennzeichnet, dass** der wirkstofffreie Flächenbereich (4) einen Anteil von 10 bis 50%, vorzugsweise 10 bis 40%, weiter vorzugsweise 20 bis 30% der Gesamtfläche der Filmzubereitung einnimmt.

3. Filmzubereitung nach Anspruch 1 oder 2, **dadurch gekennzeichnet, dass** ihre Gesamtfläche 1 bis 10 cm² beträgt.

4. Filmzubereitung nach einem der Ansprüche 1 bis 3, **dadurch gekennzeichnet, dass** der wirkstoffhaltige Flächenbereich (3) und der wirkstofffreie Flächenbereich (4) aus zwei unterschiedlichen Filmen bestehen, die aneinander gefügt sind.

5. Filmzubereitung nach Anspruch 4, **dadurch gekennzeichnet, dass** die Filmbildner der zwei unterschiedlichen Filme identisch sind.

6. Filmzubereitung nach Anspruch 4, **dadurch gekennzeichnet, dass** sich die Filmbildner der zwei unterschiedlichen Filme unterscheiden.

7. Filmzubereitung nach einem der Ansprüche 1 bis 6, **dadurch gekennzeichnet, dass** die Filmbildner ausgewählt sind aus der Gruppe bestehend aus Cellulose, Cellulosederivaten, Acryl- und Methacrylsäurepolymeren, Polyvinylalkoholen, Polyvinylacetat, Polyvinylpyrrolidon und Derivaten, Polysacchariden und Polymeren auf Stärkebasis.

8. Filmzubereitung nach einem der Ansprüche 1 bis 7, **dadurch gekennzeichnet, dass** der wirkstoffhaltige Flächenbereich (3) und der wirkstofffreie Flächenbereich (4) optisch unterscheidbar ausgebildet sind.

9. Verfahren zur Herstellung einer Filmzubereitung nach einem der Ansprüche 1 bis 7, **dadurch gekennzeichnet, dass** ein wirkstofffreier Film an einen wirkstoffhaltigen Film angefügt wird.

10. Verfahren nach Anspruch 8, **dadurch gekennzeichnet, dass** das Anfügen thermisch, durch ein Adhäsiv oder durch Feuchtigkeit erfolgt.

11. Verfahren nach Anspruch 8, **dadurch gekennzeichnet, dass** der wirkstofffreie Flächenbereich (4) durch Filmbildung in situ an den wirkstoffhaltigen Film angefügt wird.

12. Verfahren nach Anspruch 10, **dadurch gekennzeichnet, dass** die Filmbildung in situ eine Extrusion und/oder Verpressung umfasst.
